# EUROPÄISCHE PATENTSCHRIFT

(11) **EP 1 664 307 B1**
(45) Veröffentlichungstag und Bekanntmachung des Hinweises auf die Patenterteilung: **11.03.2009**
(21) Anmeldenummer: 04764721.9
(22) Anmeldetag: 02.09.2004
(51) Int. Cl.: C12N 15/82, C12N 15/11, A01H 5/10

(54) **NEUER ENDOSPERMSPEZIFISCHER PFLANZENPROMOTOR FÜR KULTURPFLANZEN**
NOVEL ENDOSPERM-SPECIFIC PLANT PROMOTER FOR CULTIVATED PLANTS
NOUVEAU PROMOTEUR DE PLANTE SPECIFIQUE DE L'ENDOSPERME POUR PLANTES CULTIVEES

(30) Priorität: 11.09.2003 DE 10343326
(43) Veröffentlichungstag der Anmeldung: 07.06.2006
(73) Patentinhaber: Maltagen Forschung GmbH, 56626 Andernach (DE)
(72) Erfinder: STAHL, Rainer, 53179 Bonn (DE); DARGATZ, Harald, 56626 Andernach (DE); LÜHRS, Renate, 53227 Bonn (DE); BERKEMEYER, Matthias, 8010 Graz (AT)
(74) Vertreter: Ahrens, Gabriele
(86) Internationale Anmeldenummer: PCT/EP2004/009762
(87) Internationale Veröffentlichungsnummer: WO 2005/026366

(56) Entgegenhaltungen:
- DATABASE EMBL 8. November 2002 (2002-11-08), ZHANG, H. ET AL.: "Barley ESTs from germinating seeds - Hordeum vulgare subsp. vulgare" XP002316563 gefunden im EBI Database accession no. CA002146
- RODRIGUEZ-PALENZUELA P ET AL: "NUCLEOTIDE SEQUENCE AND ENDOSPERM-SPECIFIC EXPRESSION OF THE STRUCTURAL GENE FOR THE TOXIN ALPHA HORDOTHIONIN IN BARLEY (HORDEUM-VULGARE L)" GENE, Bd. 70, Nr. 2, 1988, Seiten 271-281, XP002316562 AMSTERDAM ISSN: 0378-1119

### SEQUENCE LISTING

<110> Maltagen GmbH
<120> Neuer endospermspezifischer Pflanzenpromotor für Kulturpflanzen
<130> 2846 0014 WO
<140>
   <141> 2003-09-11
<150> -
   <151> 2003-11-09
<160> 4
<170> PatentIn version 3.1
<210> 1
   <211> 1564
   <212> DNA
   <213> Hordeum vulgare
<220>
   <221> misc_feature
   <222> (837)..(837)
   <223> n =C,T
<400> 1
   gagctcggag gcggtcgtgc atggactgga agcagcatgc atgcatgcat gcatcgtggc     60
   ggagaggctg gccgcgtggc gtgccgcgtg atcgatgtcg ttcacgtggc ggcctcggtg    120
   catgcggcgc ccaggtatat acgcacgaga cagtgttcgc ctctggctgc ggcggtgcat    180
   gcatgccagg ttgccagggc accgggcacg agagcgtttg gtagtcggcg ggcaggtagg    240
   gcggccgccg aggtagtaga tctcggcgag ctgccctggg cgcgtggctc gtccacggcc    300
   ggcctgggct tgcatgcagg acagcgcgcg ccaggcaagc tctgcccgcc ggtaggacca    360
   cggatcgatc gagacttcga gagtgatccc agggtcaagt gcacgcacgc actcgtttag 420
   acgatggaag atcgcctcgg ccggcctctg tcgggccaat acttcgatca gatggcatgc 480
   atgcattttc atgtgggaac acatgtaatt tcgttcactg catgcttacc ttgttatcag 540
   atctgtaaga ctgctttttc tgaagattgt cagcaagttt ttgtctggct tgcaatgcgg 600
   catcgatgtt gcacttcaga acaactttga ccgatcccct attcgaccgt aaggaaggat 660
   aaattcggtt ttcttacctt acaccacgtc tagtcgatcc cctaaacata ttacacaagt 720
   aaaattatct tccatcgtcg aaaacacact tcaatctttg ctttctaaat acgctcatcc 780
   cgcgcgcgcc cctccttctc ccacgtgttt aaatcaaata tttttatgat atactcnctc 840
   cgttcttaaa tataagtctt taaagaggtt tcactagtgt actatatacg gatgtatata 900
   gatttgtaga ttcactcatt ttgtttcgta tatagactcc tagtgaaata tcttaaaaga 960
   cttatattta agaacggagg aagtatattg catagtgttt gaaattcatg cggctagaaa 1020
   ataatgttta cacagtttca attttgttca actaatataa agatactttt ttaaataaga 1080
   gcatataaag acatcattga aggagtaaaa aaaatctaca ctaactttac ttcgttgaat 1140
   cctcttaact tttaaaggat tggattggat tggatagagt tgaaacgggc ggatcgatcg 1200
   gctcgagcgg cgcagccttc cgagacctcc atgtacgtat agaccctttt tgcgaccagg  1260
   cacttgacag tttcgagacc atcgatcatc tcgtagtcca acgccccttc gtcacaactc  1320
   acaagagaac ttcggttcaa attcttgcgt ccaaaggggc gactcctctc gtcccaaagc 1380
   atccagacgc acgctgagtt ggaacaagca gctgaacaac acggccttcc agcccgacag 1440
   gtggcgacgc tacatgcagg atgcctgcca cgaacgcacc tgcatgcaag cacggcagcg 1500
   tcctcctccg gcccaaaccc cctatataag tagagagctt ctcttcctgg caacaacaac 1560
   aaca 1564
<210> 2
   <211> 208
   <212> DNA
   <213> Hordeum vulgare
<400> 2
   atggagttca agacgaaggc gaccgtgtgc gcggcgctgc tggtgctgct cctgctttcg 60
   tcctgtacgt aattaagccc gaacgggcgc tcccaaactt attatgattt gctgctgctt 120
   attgttatgg tctaacgctt gtggccggcc accgggacat gttttttcag acgacggcgg 180
   cgacggcggc gtcggcgtgg cggaggcg 208
<210> 3
   <211> 106
   <212> DNA
   <213> Hordeum vulgare
<400> 3
   gtacgtaatt aagcccgaac gggcgctccc aaacttatta tgatttgctg ctgcttattg 60
   ttatggtcta acgcttgtgg ccggccaccg ggacatgttt tttcag 106
<210> 4
   <211> 1850
   <212> DNA
   <213> Hordeum vulgare
<220>
   <221> misc
   <222> (837)..(837)
   <223> n = C , T
<220>
   <221> misc_feature
   <222> (837)..(837)
   <223> n = C , T
<400> 4
   gagctcggag gcggtcgtgc atggactgga agcagcatgc atgcatgcat gcatcgtggc 60
   ggagaggctg gccgcgtggc gtgccgcgtg atcgatgtcg ttcacgtggc ggcctcggtg 120
   catgcggcgc ccaggtatat acgcacgaga cagtgttcgc ctctggctgc ggcggtgcat 180
   gcatgccagg ttgccagggc accgggcacg agagcgtttg gtagtcggcg ggcaggtagg 240
   gcggccgccg aggtagtaga tctcggcgag ctgccctggg cgcgtggctc gtccacggcc 300
   ggcctgggct tgcatgcagg acagcgcgcg ccaggcaagc tctgcccgcc ggtaggacca 360
   cggatcgatc gagacttcga gagtgatccc agggtcaagt gcacgcacgc actcgtttag 420
   acgatggaag atcgcctcgg ccggcctctg tcgggccaat acttcgatca gatggcatgc 480
   atgcattttc atgtgggaac acatgtaatt tcgttcactg catgcttacc ttgttatcag 540
   atctgtaaga ctgctttttc tgaagattgt cagcaagttt ttgtctggct tgcaatgcgg 600
   catcgatgtt gcacttcaga acaactttga ccgatcccct attcgaccgt aaggaaggat 660
   aaattcggtt ttcttacctt acaccacgtc tagtcgatcc cctaaacata ttacacaagt 720
   aaaattatct tccatcgtcg aaaacacact tcaatctttg ctttctaaat acgctcatcc 780
   cgcgcgcgcc cctccttctc ccacgtgttt aaatcaaata tttttatgat atactcnctc   840
   cgttcttaaa tataagtctt taaagaggtt tcactagtgt actatatacg gatgtatata   900
   gatttgtaga ttcactcatt ttgtttcgta tatagactcc tagtgaaata tcttaaaaga   960
   cttatattta agaacggagg aagtatattg catagtgttt gaaattcatg cggctagaaa  1020
   ataatgttta cacagtttca attttgttca actaatataa agatactttt ttaaataaga  1080
   gcatataaag acatcattga aggagtaaaa aaaatctaca ctaactttac ttcgttgaat  1140
   cctcttaact tttaaaggat tggattggat tggatagagt tgaaacgggc ggatcgatcg  1200
   gctcgagcgg cgcagccttc cgagacctcc atgtacgtat agaccctttt tgcgaccagg  1260
   cacttgacag tttcgagacc atcgatcatc tcgtagtcca acgccccttc gtcacaactc  1320
   acaagagaac ttcggttcaa attcttgcgt ccaaaggggc gactcctctc gtcccaaagc  1380
   atccagacgc acgctgagtt ggaacaagca gctgaacaac acggccttcc agcccgacag  1440
   gtggcgacgc tacatgcagg atgcctgcca cgaacgcacc tgcatgcaag cacggcagcg  1500
   tcctcctccg gcccaaaccc cctatataag tagagagctt ctcttcctgg caacaacaac  1560
   aacagagaac acacggtcgc cttgatcaga agctctagct ttgctccatc cacctatcta  1620
   ctacctacac ccacagaagc caatggagtt caagacgaag gcgaccgtgt gcgcggcgct  1680
   gctggtgctg ctcctgcttt cgtcctgtac gtaattaagc ccgaacgggc gctcccaaac  1740
   ttattatgat ttgctgctgc ttattgttat ggtctaacgc ttgtggccgg ccaccgggac  1800
   atgttttttc agacgacggc ggcgacggcg gcgtcggcgt ggcggaggcg              1850

## Beschreibung

Die Erfindung betrifft einen neuen endospermspezifischen Pflanzenpromotor und dessen Herstellung und Verwendung.

Rekombinante Proteinexpression in Pflanzen, insbesondere in Kulturpflanzen, ist von zunehmender industrieller Bedeutung. Zudem ist es wünschenswert gewebespezifische Promotoren zur Regelung der Proteinexpression in Pflanzen bereitzustellen.
Zahlreiche verschiedene rekombinante Proteine wurden bereits in Blättern, Knollen und in Samen von verschiedenen Pflanzenarten exprimiert (Übersicht: Daniel et al., 2001, Trends in Plant Science 6: 219-226). Die höchsten Expressionsraten (3 - 5%) wurden in Samen von Getreiden erzielt.
Für die Steuerung der Transkription der Transgene in Kulturpflanzen werden Promotoren eingesetzt, die üblicherweise die Expression der Speicherproteine kontrollieren. Getreidekörner enthalten durchschnittlich 10 - 12% Protein, Gerste sogar 12 - 18%. Den Hauptanteil dieser Proteine stellen die Speicherproteine dar (mehr als 50%). Die Hauptspeicherproteine im Endosperm der meisten Getreidearten (Ausnahme Hafer, Reis) gehören zu den sogenannten Prolaminen. Bei Gerste werden diese Proteine als Hordeine bezeichnet. Man unterscheidet die A-, B-, C-, D-, und G-Hordeine.
Für verschiedene Getreidearten wurde bereits gezeigt, dass mit Hilfe endospermspezifischer Promotoren hohe Expressionsraten für rekombinante Proteine erzielt werden können. In Reis wurde z. B. mit Hilfe des Reis-Glutelinspromotors humanes Lysozym bis zu 5% (des löslichen Proteins) im Endosperm angereichert.
In Gerste sind verschiedene Speicherprotein-Promotoren bereits zur Expression von Transgenen beschrieben (Choi et al., 2003, Plant Cell Rep. 21: 1108-20). Für den D-Hordein-Promotor sind zwei Formen bekannt (WO 9803655 A2; Horvath et al., 2000, PNAS 97: 1914-1919). Entsprechend zu den Ergebnissen in Reis betrug der Anteil des rekombinanten Proteins 5% des löslichen Proteins im Endosperm (Horvath et al., 2000, PNAS 97: 1914-1919). Daher sind im Stand der Technik Speicherprotein-Promotoren als endospermspezifisch beschrieben, da der Fachmann davon ausgehen konnte, dass solche Speicherproteine eine hohe Transkriptionsrate im Endosperm aufweisen. Des Weiteren ist beschrieben, dass solche Speicherprotein-Promotoren mittels Transkriptionsfaktoren zur Überexpression gebracht werden können (Yang et al. 2003,98: 11438-11443; WO01/83792), ebenfalls mittels synthetischer Sequenzen des Weizen- Glutelinpromotors (Norre et al. 2002, Plant Mol. Biol. 699-712 ; WO01/23593). Im genannten Stand der Technik macht der Anteil des rekombinanten Proteins im Samen nur 5% des Gesamtproteins, bzw. 0,1 % des Trockengewichtes aus.

Zhang et al "Barley ESTs from germinating seeds - Hordeum vulgare subsp. Vulgare" offenbart in der EMBL-Datenbank die Sequenz CA002146, die zum Hordothionin -Gen analog ist, jedoch keine weitere Funktion offenbart.

Überraschender Weise konnte nunmehr gezeigt werden, dass das gamma-Hordothionin-Gen eine endospermspezifische Promotoraktivität in Kulturpflanzen aufweist. Hordothione sind bisher als Pflanzen-Defensine zur Pathogenabwehr beschrieben.

Aufgabe der vorliegenden Erfindung ist es daher, einen endospermspezifischen Promotor bereitzustellen, der die gentechnische Herstellung von gewünschten Proteinen im Endosperm von Pflanzen in großen Mengen ermöglicht.

Daher ist ein Gegenstand der Erfindung ein Promotor auf der Basis des gamma-Hordothionin Gens und zwar vorzugsweise gemäß SEQ ID No. 1 mit einer Nukleinsäuresequenz 1-1564 bp, insbesondere .
a.) wenigstens ein Fragment aus 150 Nukleotiden, vorzugsweise wenigstens 500, besonders bevorzugt wenigstens 1000 Nukleotide aus der SEQ ID NO: 1,
b.) wenigstens ein Fragment aus 200 Nukleotiden, vorzugsweise wenigstens 500, besonders bevorzugt wenigstens 1000 Nukleotide aus der SEQ ID No. 1, die zu wenigstens 60%, vorzugsweise wenigstens 70%, besonders bevorzugt wenigstens 80 % homolog sind, wobei das Fragment aus 200 Nukleotiden aus der Seq ID NO. 1 wenigstens 90% homolog ist.
c.) Promotoraktivität aufweisende Fragmente der in a.) oder b.) definierten Polynukleotide.

Allesamt nachstehend erfindungsgemäße(r) Promotor(en) oder erfindungsgemäße Polynukleotide genannt.

Als besonderer Vorteil der erfindungemäßen Promotoren ist zu nennen, dass sie nicht nur während der Kornfüllung sehr aktiv sind, sondern auch während der Kornweiche. Dies ermöglicht sowohl das reifende Korn als "Bioreaktor" zu nutzen als auch die ersten Schritte eines industriellen Verfahrens, wie das Mälzungsverfahren oder Fermentationsverfahren, für die Herstellung rekombinanter Proteine einzusetzen und ebenfalls nach einer Ernte der Pflanze die gewünschten Proteine im Endosperm anzureichern.

In einer weiteren bevorzugten Ausführungsform enthalten die erfindungsgemäßen Polynukleotide zusätzlich eine Signalsequenz des Gamma-Hordothionin-Gens und zwar SEQ ID No. 2 oder hinreichende Teile davon, die den Import der Proteine in das Endoplasmatische Reticulum steuert, welches in einer weiteren bevorzugten Ausführungsform ein Intron bzw. eine nicht-codierende Sequenz und zwar SEQ ID NO: 3 oder hinreichende Teile davon enthält. Eine bevorzugte Ausführungsform dieser Kombination ist SEQ ID NO: 4. Das Intron bzw. diese nicht-codierende Sequenz hat die Funktion die Transkripte von Transgenen zu stabilisieren. Bei der Herstellung von (Transformations)Vektoren verhindert das Intron die unbeabsichtigte Expression eines rekombinanten, etwaig toxischen Proteins im Agrobacterium gemäß der Ti-Plasmid Methode (siehe Literatur unten). Zusätzlich kann dieses Signal dazu benutzt werden, das Transkript eines jeden heterologen Proteins mit einem Intron zu versehen, ohne dabei die das Intron flankierenden Sequenzen verändern zu müssen. Dies hat den Vorteil, das auf jeden Fall gewährleistet ist, dass das Intron korrekt deletiert/gespalten wird.
Mit Hilfe der Gamma-Hordothionin-Signalsequenz können rekombinante Proteine auch im Endoplasmatischen Reticulum angereichert werden oder sekretiert werden.

Vorzugsweise kann das erfindungsgemäße Polynukleotid zur Herstellung transgener monokotyler Pflanzen, insbesondere solcher der Familie Poaceae z.B. der Gattungen Triticum, Hordeum, Avena, Secale, Oryza, Zea oder Saccharum, oder der Familie Musaceae z.B. der Gattung Musa, oder der Familie Arecaceae z.B. der Gattungen Phoenix, Elaeis oder Cocös verwendet werden. Insbesondere sind die Kulturpflanzen Gerste, Weizen, Hafer, Roggen, Reis, Mais, Zuckerrohr erfindungsgemäß bevorzugt.

Ein weiterer Gegenstand der Erfindung sind ferner Polynukleotide, die erhältlich sind durch Absuchen einer DNA-oder EST-Bank mit einer entsprechenden Gensonde, z.B. bestehend aus wenigstens 150-200 Nukleotiden der erfindungsgemäßen Polynukleotide.

Solche DNA-Banken sind dem Fachmann ohne weiteres zugänglich: Gegenstand der Erfindung sind ferner Fragmente der mittels einer genannten Gensonde aufgefundenen Polynukleotide, die eine weitgehende endospermspezifische Promotoraktivität zeigen und daher unter oben c.) fallen. Bevorzugt liegt die Mindestlänge solcher Promotoraktivität aufweisender Fragmente bei 150-200 Nukleotiden.

Erfindungsgemäß wird somit ein Polynukleotid mit der biologischen Funktion eines Promotors bereitgestellt, das ein funktionell verbundenes Fremdgen in transgenen Pflanzen weitgehend endospermspezifisch exprimiert. Man kann so bestimmte Polypeptide spezifisch im Endosperm anreichern.

Der Begriff "funktionell verbunden" bedeutet, daß eine regulatorische Sequenz wie ein Promotor die Expression eines Gens steuert.

Der Begriff "transgene Pflanze" betrifft Pflanzen, die mittels rekombinanter Gentechnik und/oder mikrobiologischen Verfahren und nicht mittels herkömmlicher Züchtungsverfahren hergestellt wurden und mindestens einen erfindungsgemäßen Promotor enthalten. Verfahren zur Herstellung transgener Pflanze sind beschrieben (Tingay S., McElroy D., Kalla R., Fieg S., Wang M., Thorton S. and Brettel R. (1997): Agrobacterium tumefaciens-mediated barley transformation. Plant Journal 11; 1369 - 1376; Wan Y. and Lemaux P. (1994): Generation of a large number of independently transformed fertile barley plants. Plant Physiol. 104; 37 - 48, Stahl R., H.Horvath, J. Van Fleet, M.Voetz, D. von Wettstein & N.Wolf (2002) T-DNA integration into the barley genome from single and double cassette vectors. Proc. Natl. Acad. Sci. USA 99, 2146-2151; Horvath H., J.Huang, O.T. Wong & D.von Wettstein (2002) Experiences with genetic transformation of barley and characteristics of transgenic plants. In: Barley Science. G.A. Slafer, J.L. Molina-Cano, R. Savin, J.L. Araus & I. Romagosa eds. The Harworth Press, New York 2002 pp. 143-176; Horvath H., L.G. Jensen, O.T. Wong, E. Kohl, S.E. Ullrich, J. Cochran, C.G. Kannangara & D. von Wettstein (2001) Stability of transgene expression, field performance and recombination breeding of transformed barley lines. Theor. Appl. Genet. 102, 1-11; Wettstein D. von, G. Mikhaylenko, J.A. Froseth & C.G. Kannangara (2000) Improved barley broiler feed with transgenic malt containing heat-stable (1,3-1,4)-glucanase. Proc. Natl. Acad. Sci. USA 97, 13512-13517; Horvath H., J. Huang, O.T. Wong, E. Kohl, T. Okita, C.G. Kannangara & D.von Wettstein (2000) The production of recombinant proteins in transgenic barley grains. Proc. Natl. Acad. Sci. USA 97, 1914-1919; Mayerhofer, R. , Koncz-Kalman, Z. , Nawrath, C. , Bakkeren, G. , Crameri, A. , Angelis, K. , Redei, G. P. , Schell, J. , Hohn, B. & Koncz, C. (1991) EMBO J. 10, 697-704 T-DNA integration: a mode of illegitimate recombination in plants; Deblaere R., Bytebier B., De Greve H., Deboeck F., Schell M., Van Montagu M., Leemans J.;"Efficient octopine Ti plasmid-derived vectors for Agrobacterium-mediated gene transfer to plants"; Nucleic Acids Res. 13:4777-4788(1985)).

Der Begriff "Vektor" bezeichnet natürlich vorkommende oder künstlich erschaffene Konstrukte zur Aufnahme, Vermehrung, Expression oder Übertragung von Nukleinsäuren, z.B. Plasmide, Phagemide, Cosmide, künstliche Chromosomen, Bakteriophagen, Viren, Retroviren und mindestens einen erfindungsgemäßen Promotor enthalten. Geeignete Vektoren sind beispielhaft beschrieben in J.Sambrook, E. F. Fritsch, T. Maniatis (1989) Cold Spring Harbor Laboratory Press, Molecular Cloning A Laboratory Manual Second Edition.

Der Begriff "Homologe" oder "homologe Sequenzen" bezeichnen Nukleinsäuresequenzen mit signifikanter Ähnlichkeit zur -Vergleichssequenz oder Teilen davon. Ein unabhängig von dem Grad der Homologie anzuwendendes Kriterium besteht darin, ob ein Polynukleotid als Einzelstrang mit einem Einzelstrang entsprechender Länge aus der SEQ ID NO: 1 unter stringenten Bedingungen hybridisieren kann. Als homologe Sequenzen gelten daher Nukleinsäuresequenzen, die mit den Vergleichssequenzen oder Teilen dieser Sequenzen unter stringenten oder ggfs. wenig stringenten Bedingungen hybridisieren (zu stringenten und wenig stringenten Bedingungen siehe Sambrook et al., Molecular Cloning, Cold Spring Harbour Laboratory (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur. Als homologe Sequenzen sollen des weiteren Nukleinsäuresequenzen oder Teile davon gelten, die unter Zuhilfenahme des Similaritätsalgorithmus BLAST (Basic Local Alignment Search Tool, Altschul et al., Journal of Molecular Biology 215, 403-410 (1990) eine signifikante Ähnlichkeit mit einer Vergleichssequenz aufweisen. Als signifikant ähnlich werden, wie hier verwendet, Sequenzen bezeichnet, die z.B. unter Verwendung von Standardparametern im BLAST -Service des NCBI eine Identität von mindestens 60% aufweisen, wenn Sie mit der Vergleichssequenz verglichen werden, d.h. sie sind dann zu mindestens 60% homolog. Die erfindungsgemäßen Polynukleotide sind zum einen funktionell durch das Merkmal definiert, daß sie in Pflanzen die endospermspezifische Expression eines Fremdgens (im weiteren auch Transgen genannt) erlauben.

Der Begriff "Weitgehend" bedeutet im Sinne der Erfindung, daß die Expression des Transgens im Endosperm eine etwaige Expression gegenüber anderen Geweben/Organe der Pflanze deutlich überwiegt. Die Expression im Endosperm überwiegt im Sinne der Erfindung deutlich, wenn sie gegenüber anderen Geweben/Organen mindestens doppelt so hoch ist. Die Promotoraktivität kann sich jedoch auch über das gesamte Endosperm ohne Beschränkung auf einzelne Bereich oder Gewebe erstrecken. Im Rahmen der Erfindung ist es möglich, daß ein erfindungsgemäßes Polynukleotid als Promotor eine unspezifische Phase beispielsweise zu Beginn der Entwicklung einer transgenen Pflanze aufweist, in der die Promotoraktivität nicht auf das Endosperm beschränkt ist.

Der Begriff "Fremdgen" bedeutet im Sinn der Erfindung, daß sowohl endogene als auch exogene für ein Genprodukt codierende Nukleinsäuresequenzen verwendet werden können. Endogen bedeutet, daß die Nukleinsäuresequenz aus dem gleichen Organismus stammt, in den sie mit dem erfindungsgemäßen Verfahren integriert wird. Exogen hingegen bedeutet, daß die Nukleinsäuresequenz aus einem anderen Organismus stammt.

Die spezifisch im Endosperm hergestellten und/oder angereicherten und gegebenenfalls isolierten Polypeptide können dabei aus einem beliebigen Organismus wie z.B Mensch, Tier, Pflanze, Pilz, Bakterium, Protozoen oder Virus stammen und jedes beliebige Polypeptid sein, insbesondere werthaltige Proteine, wie therapeutische Proteine oder pharmazeutische Proteine, Antikörper oder Polypeptide, die das Wachstum der Pflanze beeinflussen können z.B. Wachstumsfaktoren, Pflanzenhormone, Hemmstoffe oder Enzymen des sekundären Stoffwechsels sein.

Bei der Einschleusung eines derartigen Promotors in die zu modifizierenden Pflanzen wird in der Regel lediglich die Expression des fusionierten Fremdgens beeinflusst. Es sind keine pleiotropen Promotoreffekte zu erwarten. Die Leistungsfähigkeit des Zuchtmaterials der betroffenen Kulturpflanze bleibt somit unberührt, soweit sie nicht durch die gewünschte Expression des Fremdgens beeinflusst wird.

Bevorzugte erfindungsgemäß verwendbare Polynukleotide sind angegeben in SEQ ID NO: 1 in Kombination mit SEQ ID NO:2 oder SEQ ID NO:3 sowie SEQ ID NO: 4. Bevorzugt sind ferner solche Polynukleotide, die zu den vorgenannten Sequenzen zu wenigstens 60%, vorzugsweise 70%, weiter vorzugsweise 80%, weiter vorzugsweise 90% homolog sind.

Ein weiterer Gegenstand der Erfindung ist ein Vektor, auch Expressionsvektor, der zumindest in einer Pflanzenzelle das erfindungsgemäße Polynukleotid enthält, welche funktionell verbunden ist für ein Genprodukt codierenden Nukleinsäuresequenz (Fremdgen). Die Erfindung betrifft daher Pflanzenzellen oder Protoplasten, die einen solchen Vektor oder das stabil in das Genom integrierte erfindungsgemäße Polynukleotid und das funktionell verbundene Fremdgen enthalten, sowie transgene Pflanzen, die solche Pflanzenzellen enthalten. Erfindungsgemäße transgene Pflanzen können monokotyle Pflanzen der vorstehend aufgeführten Familien und Gattungen sein.

Es werden erfindungsgemäß transgene Pflanzen, Pflanzenzellen oder Protoplasten mit mindestens einem nach seiner Transformation stabil in das Genom integrierten erfindungsgemäßen Polynukleotid erhalten. Daher betrifft die Erfindung ebenfalls Saatgut, welches aus den transformierten Pflanzen bzw. transgenen Pflanzen erhalten wird.

Gegenstand der Erfindung ist ferner die Verwendung eines erfindungsgemäßen Polynukleotids zur endospermspezifischen Expression eines Fremdgens in einer Pflanze sowie ein entsprechendes Verfahren zur Herstellung einer transgenen Pflanze, das folgende Schritte aufweist: Fusionieren eines Fremdgens mit einem erfindungsgemäßen Polynukleotid, gegebenenfalls Herstellen eines Vektors, der das Fusionsprodukt enthält, Einbringen des Vektors oder des Fusionsprodukts in eine Pflanzenzelle oder ein Pflanzengewebe und Regenerieren der Pflanzenzelle oder des Gewebes zu einer Pflanze, insbesondere zu einer fertilen Pflanze.

Die Erfindung wird im folgenden anhand von Ausführungsbeispielen näher erläutert, ohne die Erfindung auf diese Beispiele beschränken:

### Beispiele:

### Beispiel 1 - Transiente Versuche:

Aus einem positiven Bac-Klon wurde der 5' Bereich des Gamma-Hordothionin Gens mit dem Restriktionsenzym SstI ausgeschitten und in die SstI-Restriktionsstelle von pUC18 kloniert. Nach einer anschließenden Sequenzierung des Fragments wurden Primer synthetisiert, um den Promotor ohne die Signalsequenz zu amplifizieren und in die SmaI Restriktionsstelle von pUC18 zu klonieren. Bei dieser Amplifikation wurde unmittelbar nach der SstI Restriktionsstelle am Anfang des Promoters eine HindIII Restriktionsstelle und hinter dem Promoter eine SmaI Restriktionsstelle eingefügt, indem die Sequenzen für diese Restriktionsstellen gleich bei der Synthese der Primer mit berücksichtigt wurden. Aus diesem neu entstandenen Konstrukt konnte nun das Promotorfragment mit Hilfe der Restriktionsfragmente HindIII und SmaI in ein anderes Konstrukt eingesetzt werden, dass bereits die Sequenzen für Glucuronidase, sowie den Nos Terminator enthält. Das Resultat ist ein pUC18 Klon der das Glucuronidase Gen unter Kontrolle des Gamma-Hordothionin Promoters und des Nos Terminators enthält. Mit diesem Konstrukt wurden die Tests zur Detektion der einlagerungsspezifischen Aktivität als auch der keimungsspezifischen Aktivität durchgeführt.

### a) Einlagerungsspezifische Aktivität (endospermspezifische Aktivität)

Es wurde Endospermgewebe (ca.20 Tage nach der Befruchtung) isoliert und auf Nährmedium CIM(0) gelegt.

| CIM(0) : | |
|---|---|
| M5524 (MS salts, Sigma) | 4,30g |
| Maltose | 30,00g |
| Myo-Inositol | 0,25g |
| Casein Enzymatic Hydrolysate | 1,00g |
| L-Proline | 0,69g |
| Thiamin-HCL (4mg/ml) | 250µl |
| CuSO4 (50 mM) | 100µl |
| H₂O hinzufügen auf | 1000µl |

Anschließend pH-Wert mit NaOH auf 5,8 einstellen und 3,5 g Phytagel zufügen. Dann 20 Minuten autoklavieren und 250 µl Dicamba/l Medium (stock 10 mg/ml in DMSO) zufügen.

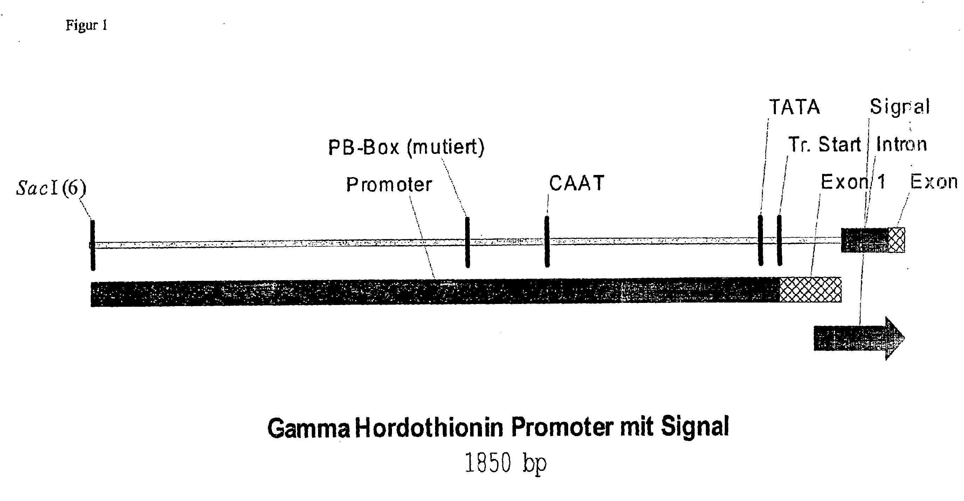

Nach 2 Tagen wurde das Endospermgewebe mittels Gold-Partikeln mit Hilfe einer "Particle-Gun" beschossen. Hierzu wurde 1,25 mg Goldstaub in 100 µl Ethanol aufgenommen und durch vortexen (rütteln) gewaschen. Diese Prozedur wurde einmal wiederholt. Anschließend wurde das Gold in 250 ml Wasser aufgenommen und mit einem DNA-Konstrukt versehen, das den Gamma-Hordothionin-Promoter mit Signal-Peptid plus Glucuronidase plus dem Nos Terminator enthält (z.B. gemäß Figur 1 oder SEQ ID No: 4). Dieses Konstrukt befindet sich in pUC18 als Vektor. 8 µl dieser DNA (Konz. 1 µg/µl) wurden zu dem in Wasser aufgenommenen Gold gegeben. Anschließend wurden 250 µl CaCl2 (Stock Lösung 2,5 M) und 100 µl Spermidin zu der DNA-Goldmischung gegeben. Das ganze wurde 3 min. gevortext (gerüttelt) und 10 min. bei 10000 u/min. abzentrifugiert. Nachdem der Überstand abgenommen wurde, wurden je 2 µl der Gold-DNA Mischung auf Macrocarriern verteilt und mit.Hilfe der "Particle Gun" auf die 2 Tage alten Endosperme geschossen. Nach einer Inkubation von 2 Tagen bei 24°C wurd mit Hilfe eines Glucuronidase Tests die Aktivität durch blaue Spots sichtbar.

### Beispiel 2 - Stabile Transformation:

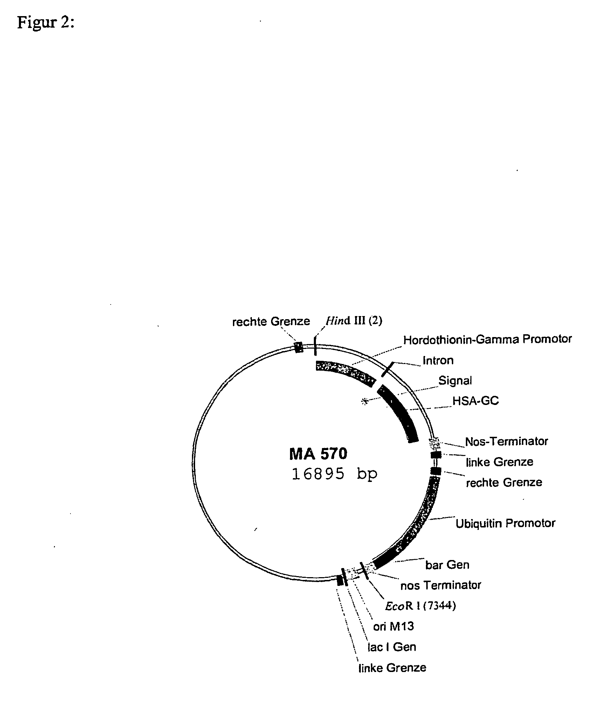

Mittels einer PCR wurde der Gamma-Hordothionin-Promoter samt der Sequenz des Signal Peptids mit DNA aus dem positiven Bac-Klon amplifiziert. Dabei wurden durch entsprechendes Primerdesign die ersten 20 Basepaare des reifen codon-optimierten humanen Serumalbumin direkt hinter das Signalpeptid des Gamma Hordothioninpromotors angefügt. In einer weiteren PCR wurden mittels "Splice by Overlapp" die ersten 400 Basepaare des reifen codon - optimierten humanen Serumalbumin angehangen, die eine PstI Restriktionsstelle beinhalten. Das Amplifikat wurde wiederum in die SmaI Restriktionsstelle von pUC18 kloniert. Aus diesem Konstrukt wurde dann mit HindIII/PstI der Gammahordothioninpromotor samt Signalpeptid mit dem ersten Teil des reifen codon - optimierten humanen Serumalbumin ausgeschnitten und in MA 67 kloniert, aus dem mit den gleichen Enzymen der gamma Hordithionin Promoter mit dem ersten Teil des reifen codon - optimierten humanen Serumalbumin ausgeschnitten wurde. Anschließend wurde aus diesem Konstrukt mit EcoRI samt Auffüllen mit T4 Polymerase und HindIII das gesamte Gammahordothionin-Signal-HSA-Nos Konstrukt ausgeschnitten und in MA 185 kloniert, aus dem mit BamHI samt Auffüllen mit T4 Polymerase und HindIII ein kleines Stück ausgeschnitten wurde. Dieser Klonierungsschritt hatte den Sinn das Gammahordothionin-Signal-HSA-Nos-Fragment mit einer Kassette zusammenzuklonieren, die eine Selektion des transformierten Gewebes mit Bialaphos zulässt. Aus diesem Konstrukt wiederum wurde mit EcoRI und HindIII das Gammahordothionin-Signal-HSA-Nos-Fragment mit der Selektionskassette ausgeschnitten und gegen ein gleich geschnittenes Stück in MA128 ausgetauscht. Das entstandene Konstrukt trägt den Namen MA 570 (siehe Figur 2).
Mit dem binären Transformationsvektor MA 570 wurden Agrobakterien vom Stamm AglI transformiert, die wiederum zur Transformation von unreifen Gerstenembryonen benutzt wurden. Diese wurden aus dem Kultivar Golden Promise isoliert und nach 2 Tagen Inkubation auf CIM(o) mit den Plasmid tragenden Agrobakterien für 2 Tage co-kultiviert.

| CIM(0) : | |
|---|---|
| M5524 (MS salts, Sigma) | 4,30g |
| Maltose | 30,00g |
| Myo-Inositol | 0,25g |
| Casein Enzymatic Hydrolysate | 1,00g |
| L-Proline | 0,69g |
| Thiamin-HCL (4mg/ml) | 250µl |
| CuSO4 (50 mM) | 100µl |
| H₂O hinzufügen auf | 1000µl |

Anschließend pH-Wert mit NaOH auf 5,8 einstellen und 3,5 g Phytagel zufügen. Dann 20 Minuten autoklavieren und 250 µl Dicamba/l Medium (stock 10 mg/ml in DMSO) zufügen.

Danach wurden die Agrobakterien abgewaschen und die transformierten Embryonen auf Selektivmedium CIM(4) umgesetzt. Nach 2 Wochen werden die Embryonen auf neue CIM(4)Platten gesetzt. Dies wurde wiederholt nach weiteren 2 Wochen.
CIM(4): wie CIM(0), nur mit 4 mg/l Bialaphos + 200 µg/l Timentin.
Nach weiteren 2 Wochen wurden die Embryonen auf FHG-Medium umgesetzt.

| FHG: | |
|---|---|
| M2909 (MS salts, Sigma) | 2,7g |
| Maltose | 62,0g |
| Myo-Inositol | 0,1g |
| Casein Enzymatic Hydrolysate | 1,0g |
| NH4NO3 | 165.0 mg |
| Thiamin-HCL (4mg/ml) | 100µl |
| CuSO4 (50 mM) | 100µl |
| H₂O hinzufügen auf | 1000µl |

Anschließend pH-Wert mit NaOH auf 5,6 einstellen und 3,5 g Phytagel zufügen. Dann 20 Minuten autoklavieren 300 µl Bialaphos/l Medium (stock 10 mg/ml), 1 ml Timentin (stock 200 mg/ml) und 400 µl BAP (stock 2,5 mg/ml) hinzufügen.

Auf diesem Medium regenerierten sich transgene Pflanzen, die sowohl für das Humane Serum Albumin als auch für den Selektierungsmarker, das Bar Gen aktiv sind.

### Beispiel 3 - Funktionstest für das Hordothionin Signal

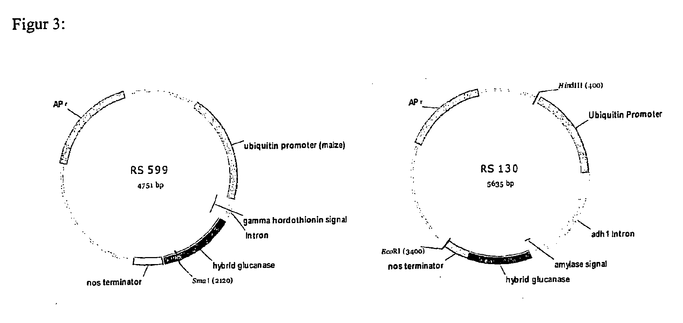

Peptid mit Intron unter einem konstitutivem Promoter. Dieser Test wurde transient in Aleuronzellen unter Kontrolle des Ubiquitin Promoters aus Mais ausgeführt. Mittels PCR wird aus einem Klon der die Sequenz für das Hordothionin Signal incl. Intron enthält, dieses amplifiziert. Durch diese PCR wurde vor dem Hordothionin Signal Peptid ein Stück der 3' Sequenz des Ubiquitin Promoters aus Mais und hinter dem Signal Peptid ein Stück der 5' Sequenz der reifen Hybrid-Glucanase fusioniert. In einer weiteren PCR wurde ein weiteres Stück des 3' Bereiches des Ubiquitin Promoters vor das zuvor generierte PCR-Fragment fusioniert, so dass sich nun die Sequenz des 3' Bereichs des Ubiquitin Promoters bis einschließlich der BglII Restriktionsstelle vor der Sequenz des Hordothionin Signal befindet und dahinter die ersten 21 Nukleotide der reifen Hybrid-Glucanase. Dieses PCR Fragment wurde mittels "Splice by Overlapp" mit einem weiteren PCR Fragment fusioniert, das aus der Sequenz der reifen Hybrid-Glucanase besteht. Dieses PCR Produkt wurde in die SmaI Restriktionstelle von pUC18 kloniert. Hieraus wiederum wurde mit BglII/ SmaI ein Fragment ausgeschnitten und in einen ebenfalls BglII/SmaI geschnittenen Klon kloniert der den Ubiquitin Promoter, das adh1 Intron, das Amylase Signal sowie die reife Hybrid Glucanase und dem Nos Terminator beinhaltet. Das adh1 Intron und das Amylase Signalpeptid wurden hierbei durch die Sequenz des ein Intron enthaltenden Gamma Hordothionin Signalpeptids ausgetauscht (siehe Figur 3).

Dieses Konstrukt wird in Aleuronprotoplasten in einem Transiententest getestet.
Mit Aleuron Protoplasten der Sorte Himalaya wurden transiente Versuche durchgeführt. Bei den Körnern dieser Sorte wurde mit Hilfe eines Skalpells der Embryo, sowie ein Stück des distalen Ende entfernt. Anschließend wurde das Korn in der Länge (genau in der Furche) geteilt. Daraufhin wurden die Körner in 2 ml Wasser für 10-15 min. eingeweicht. Eine Sterilisation erfolgte durch Zugabe von 4 ml Sterilisationslösung (1 ml Natriumhypochlorid plus 49 ml steriles Wasser). Danach wurde die Körner 4 Mal mit sterilem Wasser gewaschen.und in sterile Petrischalen überführt. Anschließend wurden die Körner in je 10 ml einer 0,015 % igen Cefotaximelösung für 60 Stunden bei Raumtemperatur eingeweicht. Nach dieser Inkubationszeit wurde die Stärke entfernt und die Außenhäute mit der anhaftenden Aleuronschicht für 15 Stunden in einer 3 % - igen Cellulaselösung in APIM inkubiert.
APIM:

| | |
|---|---|
| 3,1 g Gamborgs B5 Basal Salt Mixture auf einen Liter | |
| 2% Glucose | |
| L-Arginine | 10 mM |
| Cac12 X 2H2O | 20 mM |
| MES | 10 mM |
| Mannitol | 300 mM |
| pH 5,4, 600 mOs | |

Hierbei wurden die Aleuronzellen (Protoplasten) von der Außenhaut abgelöst. Über je 5 ml eines Percoll Gradienten (9 ml ATP und 11 ml Percoll) wurden die Aleuronprotoplasten aufgereinigt.

| | |
|---|---|
| Mannitol | 400 mM |
| MgCl2 X 6H2O | 15 mM |
| MES | 5 mM |
| Glucose | 2% (w/v) |

Für eine Transfektion-wurden ca. 600.000 Protoplasten benötigt. Diese wurden nun mittels PEG 3350 mit 150 µg DNA transfektiert und für 20 min bei Raumtemperatur inkubiert. Danach wurden in 3 Minuten Intervallen je. 2 ml Aliquots von 0,2M CaC12 zugegeben. Als Kontrolle dienten transfektierte Aleuronzellen, die mit einem Konstrukt transfektiert wurden, das den Ubiquitin Promoter, gefolgt von dem adh1 Intron aus Mais, dem-Amylase Signal Peptid, der reifen Hybrid-Glucanase sowie dem Nos Terminator enthält.

### Ergebnis:

Mit Hilfe der Nukleotidsequenz des Gamma Hordothionin Signal Peptids incl. des Introns lässt sich die Expression von Proteinen im Gegensatz zur Vewendung der Nukleotidsequenz des adh1 Intons aus Mais und dem Amylase Signals in Aleuronzellen um den Faktor 4-5 steigern. Beide Introns, bzw. Signalsequenzen wurden unter Kontrolle des Ubiquitin Promoters aus Mais benutzt. Das Reportergen ist bei beiden Versuchen die Hybrid-Glucanase.

Je 50 µl von 1 ml Protoplastenüberständen wurden für einen Glucanase-Aktivitäts-Test eingesetzt.

| | | |
|---|---|---|
| | | Ubiqutin |
| | Ubiquitin | Promoter- |
| | Promoter- | Hordothionin |
| | adh1 | Signal |
| | Intron- | (Intron)- |
| | Amylase | Hybrid |
| | Signal-Nos | Glucanase-Nos |
| 1 | 0,192 | 0,965 |
| 2 | 0,228 | 0,878 |
| 3 | 0,173 | 0,974 |

### Beschreibung der Figuren:

Figur 1: Zeigt den 5' Bereich des Gamma-Hordothionin Gens (Promoter+Signal) mit einer geringfügig mutierten Prolamin-Bindungs-Box (tttaaag) für den Transkriptionsfaktor BPBF (Barley Prolamin Binding Factor) Die übliche Sequenz der Prolamin Bindungsboxen in Gerste ist: tgtaaag.

Figur 2: Binärer Doppelkassetten-Transformationsvektor für Agrobakterien und Pflanzen. Zwischen der ersten linken und rechten border befindet sich eine Kassette aus Gamma-Hordothionin-Promoter, Signalpeptid und NosTerminator, zwischen der anderen linken und rechten border befindet sich der Mais-Ubiquitinpromoter mit dem 1. Intron, das Bar-Gen und der Nos Terminator.

Figur 3: Eingesetzte Transformationsvektoren gemäß Beispiel 3.

## Patentansprüche

1. Polynukleotid, das in Pflanzen eine weitgehend endospermspezifische Expression eines Fremdgens erlaubt, ausgewählt aus der Gruppe bestehend aus:
a.) wenigstens ein Fragment aus 150 Nukleotiden aus der SEQ ID NO: 1,
b.) wenigstens ein Fragment aus 200 Nukleotiden aus der SEQ ID NO: 1, die zu wenigstens 90% homolog sind,
c.) Promotoraktivität aufweisende Fragmente der in a.) oder b.) definierten Polynukleotide.

2. Polynukleotid nach Anspruch 1, umfassend zusätzlich eine Signalsequenz wie SEQ ID NO: 2 oder Teile davon oder eine nicht-codierende Sequenz SEQ ID NO: 3 oder Teile davon, oder SEQ ID NO: 4 und Polynukleotiden, die zu den vorgenannten Sequenzen zu wenigstens 60% homolog sind.

3. Vektor, enthaltend ein Polynukleotid gemäß Anspruch 1 oder 2.

4. Transgene Pflanze mit mindestens einem nach seiner Transformation stabil in das Genom integrierten Polynukleotid nach Anspruch 1 oder 2, und einer mit dem Polynukleotid funktionell verbundenen für ein Genprodukt codierenden Nukleinsäuresequenz, oder mit einem in den Pflanzenzellen vorliegenden Vektor nach Anspruch 3.

5. Transgene Pflanze nach Anspruch 4, wobei die Pflanze ausgewählt ist aus monokotylen Pflanzen, vorzugsweise der Familie Poaceae, insbesondere der Gattungen Triticum, Hordeum, Avena, Secale, Oryza, Zea oder Saccharum, oder der Familie Musaceae, insbesondere der Gattung Musa, oder der Familie Arecaceae, insbesondere der Gattungen Phoenix, Elaeis oder Cocös oder Kulturpflanzen, wie Gerste, Weizen, Hafer, Roggen, Reis, Mais, Zuckerrohr.

6. Transformierte Pflanzenzelle oder transformiertes Pflanzengewebe oder transformierter Protoplast mit einem Vektor gemäß Anspruch 3 oder mit einem nach seiner Transformation stabil in das Genom integrierten Polynukleotid gemäß Anspruch 1 oder 2 und einer mit dem Polynukleotid funktionell verbundenen für ein Genprodukt codierenden Nukleinsäuresequenz.

7. Transformierte Pflanzenzelle oder transformiertes Pflanzengewebe oder transformierter Protoplast nach Anspruch 6, regenerierbar zu einer fertilen Pflanze.

8. Transgenes Saatgut, erhalten von Pflanzen nach Anspruch 4 oder 5.

9. Verwendung eines Polynukleotids gemäß Anspruch 1 oder 2 oder eines Vektors nach Anspruch 3 zur endospermspezifischen Expression eines Fremdgens in einer Pflanze.

10. Verwendung nach Anspruch 9, **dadurch gekennzeichnet, dass** die Pflanze ausgewählt ist aus monokotylen Pflanzen, vorzugsweise der Familie Poaceae, insbesondere der Gattungen Triticum, Hordeum, Avena, Secale, Oryza, Zea oder Saccharum, oder der Familie Musaceae, insbesondere der Gattung Musa, oder der Familie Arecaceae, insbesondere der Gattungen Phoenix, Elaeis oder Cocös oder Kulturpflanzen, wie Gerste, Weizen, Hafer, Roggen, Reis, Mais, Zuckerrohr.

11. Verfahren zur Herstellung einer transgenen Pflanze, umfassend die folgenden Schritte:
a.) Fusionieren eines Fremdgens mit einem Polynukleotid nach Anspruch 1 oder 2,
b.) gegebenenfalls Herstellen eines Vektors, der das Fusionsprodukt aus Schritt a.) enthält,
c.) Einbringen des Fusionsprodukts aus Schritt a.) oder des Vektors aus Schritt b.) in eine Pflanzenzelle oder ein Pflanzengewebe,
d.) Regenerieren der Pflanzenzelle oder des Gewebes zu einer Pflanze, insbesondere zu einer fertilen Pflanze.

## Claims

1. A polynucleotide allowing a largely endosperm-specific expression of a foreign gene in plants, which is selected from the group consisting of:
a) at least one fragment of 150 nucleotides from SEQ ID NO: 1,
b) at least one fragment of 200 nucleotides from SEQ ID NO: 1, having a homology of at least 90%, and
c) promoter activity-exhibiting fragments of the polynucleotide defined in (a) or (b).

2. A polynucleotide according to claim 1, additionally comprising a signal sequence selected from the group consisting of SEQ ID NO: 2 or portions thereof, or a noncoding sequence of SEQ ID NO: 3 or portions thereof, or SEQ ID NO: 4, and polynucleotides comprising homology to the aforementioned sequences of at least 60%.

3. A vector comprising a polynucleotide according to claim 1 or claim 2.

4. A transgenic plant with at least one polynucleotide according to claim 1 or 2, which polynucleotide is stably integrated into the genome of the plant after its transformation, and a nucleic acid sequence coding for a gene product, which is operatively linked to the polynucleotide, or being presented in a vector according to claim 3 within the plant cells.

5. A transgenic plant according to claim 4, wherein the plant is selected from the group consisting of monocotyle plants, preferably plants of the Poaceae family, particularly plants of the genus Triticum, Hordeum, Avena, Secale, Oryza, Zea or Saccharum, particularly plants of the Musaceae family, particularly plants of the genus Musa, or plants of the Arecaceae family, particularly plants of the genus Phoenix, Elaeis or Cocos or cultivated plants like barley, wheat, oat, rye, rice, corn, and sugarcane.

6. A transformed plant cell or transformed plant tissue or transformed protoplast comprising the vector according to claim 3 or a polynucleotide according to claim 1 or 2, which is stably integrated into the genome of the plant after its transformation, and a nucleic acid sequence coding for a gene product, which is operatively linked to the polynucleotide.

7. A transformed plant cell or transformed plant tissue or transformed protoplast according to claim 6 regenerated to a fertile plant.

8. A transgenic seed obtained from the plant of claim 4 or 5.

9. Use of a polynucleotide according to claim 1 or 2 or a vector according to claim 3 for endosperm-specific expression of a foreign gene in a plant

10. Use of claim 9, **characterized in that**, the plant is selected from the group consisting of monocotyle plants, preferably plants of the Poaceae family, particularly plants of the genus Triticum, Hordeum, Avena, Secale, Oryza, Zea or Saccharum, particularly plants of the Musaceae family, particularly plants of the genus Musa, or plants of the Arecaceae family, particularly plants of the genus Phoenix, Elaeis or Cocos or cultivated plants like barley, wheat, oat, rye, rice, corn, and sugarcane.

11. A method for the production of a transgenic plant, comprising the following steps:
(a) fusing of a foreign gene with a polynucleotide according to claim 1 or 2,
(b) optionally generating of a vector containing the fusion product of (a),
(c) introducing of the fusion product of step (a) or the vector of step (b) into a plant cell or plant tissue,
(d) regenerating the plant cell or tissue to a plant, particularly to a fertile plant.

## Revendications

1. Polynucléotide, qui permet une expression d'un gène étranger essentiellement spécifique à l'endosperme dans des plantes, choisi dans le groupe constitué par :
a.) au moins un fragment de 150 nucléotides provenant de la SEQ ID N°1,
b.) au moins un fragment de 200 nucléotides provenant de la SEQ ID N°1, qui sont homologues à au moins 90 %,
c.) des fragments des polynucléotides, définis dans a.) ou b.), présentant une activité de promoteur.

2. Polynucléotide selon la revendication 1, comprenant en plus une séquence signal comme la SEQ ID N°2 ou des parties de celle-ci ou une séquence non codante SEQ ID N°3 ou des parties de celle-ci, ou la SEQ ID N°4 et des polynucléotides, qui sont homologues à au moins 60 % avec les séquences précitées.

3. Vecteur, contenant un polynucléotide selon la revendication 1 ou 2.

4. Plante transgénique ayant au moins un polynucléotide selon la revendication 1 ou 2 intégré dans le génome de manière stable après sa transformation, et une séquence d'acide nucléique liée de manière fonctionnelle avec le polynucléotide et codant pour un produit génique, ou ayant un vecteur présent dans les cellules végétales selon la revendication 3.

5. Plante transgénique selon la revendication 4, où la plante est choisie parmi les plantes monocotylédones, de préférence la famille des Poaceae, notamment des genres Triticum, Hordeum, Avena, Secale, Oryza, Zea ou Saccharum, ou de la famille des Musaceae, notamment du genre Musa, ou de la famille des Arecaceae, notamment des genres Phoenix, Elaeis ou des cocos ou plantes cultivées comme l'orge, le blé, l'avoine, le seigle, le riz, le maïs, la canne à sucre.

6. Cellule végétale transformée ou tissu végétal transformé ou protoplaste transformé avec un vecteur selon la revendication 3 ou avec un polynucléotide selon la revendication 1 ou 2 intégré de manière stable dans le génome après sa transformation et avec une séquence d'acide nucléique liée de manière fonctionnelle avec le polynucléotide et codant pour un produit génique.

7. Cellule végétale transformée ou tissu végétal transformé ou protoplaste transformé selon la revendication 6, pouvant être régénéré en une plante fertile.

8. Semence transgénique, obtenue à partir de plantes selon la revendication 4 ou 5.

9. Utilisation d'un polynucléotide selon la revendication 1 ou 2 ou d'un vecteur selon la revendication 3 pour l'expression d'un gène étranger, spécifique à l'endosperme, dans une plante.

10. Utilisation selon la revendication 9, **caractérisée en ce que** la plante est choisie parmi les plantes monocotylédones, de préférence la famille des Poaceae, notamment des genres Triticum, Hordeum, Avena, Secale, Oryza, Zea ou Saccharum, ou de la famille des Musaceae, notamment du genre Musa, ou de la famille des Arecaceae, notamment des genres Phoenix, Elaeis ou des cocos ou plantes cultivées comme l'orge, le blé, l'avoine, le seigle, le riz, le maïs, la canne à sucre.

11. Procédé pour la production d'une plante transgénique, comprenant les étapes suivantes :
a.) la fusion d'un gène étranger avec un polynucléotide selon la revendication 1 ou 2,
b.) le cas échéant la production d'un vecteur qui comprend le produit de fusion issu de l'étape a.),
c.) l'intégration du produit de fusion issu de l'étape a.) ou du vecteur issu de l'étape b.) dans une cellule végétale ou dans un tissu végétal,
d.) la régénération de la cellule végétale ou du tissu en une plante, notamment en une plante fertile.
